(19) Europäisches Patentamt
European Patent Office
Office européen des brevets

(11) Veröffentlichungsnummer: **0 195 238**
**B1**

(12) # EUROPÄISCHE PATENTSCHRIFT

(45) Veröffentlichungstag der Patentschrift: **29.03.89**

(51) Int. Cl.⁴: **C 07 D 231/56,** A 01 N 43/56

(21) Anmeldenummer: **86101727.5**

(22) Anmeldetag: **11.02.86**

(54) **Fluorhaltige N-sulfenylierte Indazole, Verfahren zu ihrer Herstellung sowie ihre Verwendung.**

(30) Priorität: **21.02.85 DE 3505905**

(43) Veröffentlichungstag der Anmeldung: **24.09.86 Patentblatt 86/39**

(45) Bekanntmachung des Hinweises auf die Patenterteilung: **29.03.89 Patentblatt 89/13**

(84) Benannte Vertragsstaaten: **BE CH DE FR GB IT LI NL SE**

(56) Entgegenhaltungen:
**FR-A-1 568 790**
**US-A-3 786 152**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Heywang, Gerhard, Dr., Nittumer Weg 4, D-5060 Bergisch Gladbach 2 (DE)**
Erfinder: **Baasner, Bernd, Dr., Hamberger Strasse 27d, D-5090 Leverkusen 3 (DE)**
Erfinder: **Marhold, Albrecht, Dr., Carl- Duisberg-Strasse 329, D-5090 Leverkusen 1 (DE)**
Erfinder: **Paulus, Wilfried, Dr., Deswatinesstrasse 90, D-4150 Krefeld 1 (DE)**
Erfinder: **Reinecke, Paul, Dr., Steinstrasse 8, D-5090 Leverkusen 3 (DE)**
Erfinder: **Schmitt, Hans- Georg, Dr., Am Oberend 13, D-4150 Krefeld 1 (DE)**

**EP 0 195 238 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft neue, fluorhaltige N-sulfenylierte Indazole, ein Verfahren zu ihrer Herstellung sowie ihre Verwendung im Materialschutz und als Pflanzenschutzmittel.

Es ist bereits bekannt, daß bestimmte N-sulfenylierte Indazole fungizide und mikrobizide Eigenschaften aufweisen (vgl. US-A-3 867 540 und US-A 3-786 152). Die Wirkung dieser Verbindungen ist jedoch in bestimmten Indikationsgebieten, insbesondére bei niedrigen Aufwandmengen und -konzentrationen, nicht immer befriedigend.

Es wurden nun neue fluorhaltige N-sulfenylierte Indazole der allgemeinen Formel (I) gefunden,

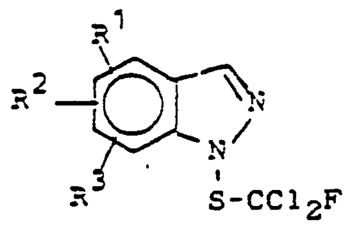
(I),

worin

$R^1$, $R^2$ und $R^3$ gleich oder verschieden sind und für Wasserstoff, Fluor, Chlor, Brom, eine Nitrogruppe, eine gegebenenfalls ein- oder mehrfach durch Fluor, Chlor oder Brom substituierte ($C_1$-$C_6$) Alkyl-, ($C_1$-$C_4$) Alkoxy- oder ($C_1$-$C_4$) Alkylthiogruppe stehen oder zwei der Substituenten $R^1$ bis $R^3$ zusammen einen 5,6-Dioxymethylen-, 5,6-Dioxy-difluormethylen-, 5,6-Dioxy-chlorfluormethylen-, 5,6-Dioxyethylen-, 5,6-Dioxy-difluorethylen-, 5,6-Dioxy-trifluorethylen-, 5,6-Dioxy-tetrafluorethylen-, 5,6-Dioxy-chlortrifluorethylen-, 5,6-Dioxy-dichlordifluorethylen-, 5,6-Dioxy-chlordifluorethylen-, 5,6-Dioxy-chlorfluorethylen- oder 5,6-Dioxy-dichlorfluorethylen-Rest bilden,

mit der Maßgabe, daß mindestens einer der Substituenten $R^1$ bis $R^3$ für Fluor steht oder ein fluorsubstituiertes Kohlenstoffatom enthält und daß, wenn einer der Substituenten $R^1$, $R^2$ oder $R^3$ für Fluor steht, höchstens einer der beiden verbleibenden Substituenten Wasserstoff ist.

Als $C_1$-$C_4$-Alkylgruppen seien bevorzugt Methyl, Ethyl, n-Propyl und iso-Propyl genannt; als $C_1$-$C_4$-Alkoxygruppen bevorzugt solche mit 1 bis 2 Kohlenstoffatomen, wie Methoxy sind Ethoxy und als $C_1$-$C_4$-Alkylthiogruppen bevorzugt solche mit 1 bis 2 Kohlenstoffatomen, wie Methylthio und Ethylthio.

Bevorzugt sind solche fluorhaltigen N-sulfenylierten Indazole der Formel (I)

wenn in dieser

$R^1$, $R^2$ und $R^3$ gleich oder verschieden sind und für Wasserstoff, Methyl, Fluormethyl, Difluormethyl, Trifluormethyl, Chlormethyl, Dichlormethyl, Trichlormethyl, Chlordifluormethyl, Difluormethyl, Methoxy, Fluormethoxy, Difluormethoxy, Trifluormethoxy, Chlordifluormethoxy, Dichlorfluormethoxy, 1,1,2,2-Tetrafluorethoxy, 2-Chlor-1,1,2-trifluorethoxy, 2,2,2-Trifluorethoxy, 2,2-Dichlor-1,1,2-Trifluorethoxy, 2-Chlor-1,1,2,2-tetrafluorethoxy, 2-Chlor-2,2-difluorethoxy, 2,2-Dichlor-2-fluorethoxy, 2,2,2-Trichlorethoxy, 1,1,2,3,3,3-Hexafluorpropoxy, Methylthio, Chlordifluormethylthio, Difluormethylthio, Trifluormethylthio stehen oder zwei der Substituenten $R^1$ bis $R^3$ zusammen einen 5,6-Dioxymethylen, 5,6-Dioxy-difluormethylen, 5,6-Dioxy-chlorfluormethylen, 5,6-Dioxy-ethylen, 5,6-Dioxy-difluorethylen, 5,6-Dioxy-trifluorethylen, 5,6-Dioxy-tetrafluorethylen, 5,6-Dioxychlortrifluorethylen, 5,6-Dioxy-dichlordifluorethylen, 5,6-Dioxy-chlordifluorethylen, 5,6-Dioxy-chlorfluorethylen Rest oder 5,6-Dioxy-dichlorfluorethylen bilden, mit der Maßgabe, daß mindestens einer der Substituenten $R^1$ bis $R^3$ für Fluor steht oder ein fluorsubstituiertes Kohlenstoffatom enthält und daß, wenn einer der Substituenten $R^1$, $R^2$ oder $R^3$ für Fluor steht, höchstens einer der beiden verbleibenden Substituenten Wasserstoff ist.

Beispielhaft seien folgende neue fluorhaltige N-sulfenylierte Indazole namentlich aufgeführt:

1-Dichlorfluormethylsulfenyl-4-trifluormethoxy-indazol,
1-Dichlorfluormethylsulfenyl-5-trifluormethoxy-indazol,
1-Dichlorfluormethylsulfenyl-6-trifluormethoxy-indazol,
1-Dichlorfluormethylsulfenyl-4-trifluormethyl-6-methyl-7-chlor-indazol,
1-Dichlorfluormethylsulfenyl-5-difluormethoxy-indazol,
1-Dichlorfluormethylsulfenyl-[2-chlor-1.1.2-trifluorethoxy]-indazol
1-Dichlorfluormethylsulfenyl-5-chlor-difluormethoxyindazol
1-Dichlorfluormethylsulfenyl-4-chlor-5-trifluormethoxyindazol,
1-Dichlorfluormethylsulfenyl-4-methyl-5-trifluormethoxyindazol,
1-Dichlorfluormethylsulfenyl-5-brom-6-fluor-indazol,
1-Dichlorfluormethylsulfenyl-5,7-dimethyl-6-trifluormethyl-indazol,
1-Dichlorfluormethylsulfenyl-6-trifluormethylthio-indazol,
1-Dichlorfluormethylsulfenyl-4,5-dichlor-6-trifluormethyl-indazol,
1-Dichlorfluormethylsulfenyl-5-dichlorfluormethylthioindazol,
1-Dichlorfluormethylsulfenyl-5-trifluormethylthioindazol,
1-Dichlorfluormethylsulfenyl-6-dichlorfluormethylthioindazol,

1-Dichlorfluormethylsulfenyl-6-difluormethoxy-indazol,
1-Dichlorfluormethylsulfenyl-5-trifluormethyl-6-chlorindazol,
1-Dichlorfluormethylsulfenyl-5-trifluormethyl-6-fluorindazol,
1-Dichlorfluormethylsulfenyl-4-trifluormethyl-6-chlorindazol,
1-Dichlorfluormethylsulfenyl-5-(2.2.2-trifluorethoxy)-indazol,
1-Dichlorfluormethylsulfenyl-5-(1.1.2.2-tetrafluorethoxy)-indazol,
1-Dichlorfluormethylsulfenyl-6-(1.1.2.2-tetrafluorethoxy)-indazol,
1-Dichlorfluormethylsulfenyl-6-(2.2.2-trifluorethoxy)-indazol,
1-Dichlorfluormethylsulfenyl-4-methyl-6-fluor-indazol.

Weiterhin betrifft die Erfindung ein Verfahren zur Herstellung der neuen fluorhaltigen N-sulfenylierten Indazole.

Es wurde ein Verfahren zur Herstellung der neuen fluorhaltigen N-sulfenylierten Indazole der Formel (I) gefunden

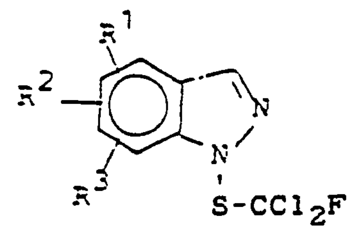

(I),

worin

$R^1$, $R^2$ und $R^3$ gleich oder verschieden sind und für Wasserstoff, Fluor, Chlor, Brom, eine Nitrogruppe, eine gegebenenfalls ein- oder mehrfach durch Fluor, Chlor oder Brom substituierte ($C_1$-$C_6$) Alkyl-, ($C_1$-$C_4$) Alkoxy- oder ($C_1$-$C_4$) Alkylthiogruppe stehen oder zwei der Substituenten $R^1$ bis $R^3$ zusammen einen 5,6-Dioxymethylen-, 5,6-Dioxy-difluormethylen-, 5,6-Dioxy-chlorfluormethylen-, 5,6-Dioxyethylen-, 5,6-Dioxy-difluorethylen-, 5,6-Dioxy-trifluorethylen-, 5,6-Dioxy-tetrafluorethylen-, 5,6 Dioxy-chlortrifluorethylen-, 5,6-Dioxy-dichlordifluorethylen-, 5,6-Dioxy-chlordifluorethylen-, 5,6-Dioxy-chlorfluorethylen- oder 5,6-Dioxy-dichlorfluorethylen-Rest bilden,

mit der Maßgabe, daß mindestens einer der Substituenten $R^1$ bis $R^3$ für Fluor steht oder ein fluorsubstituiertes Kohlenstoffatom enthält und daß, wenn einer der Substituenten $R^1$, $R^2$ oder $R^3$ für Fluor steht, höchstens einer der beiden verbleibenden Substituenten Wasserstoff ist,

das dadurch gekennzeichnet ist, daß man Indazole der Formel (V)

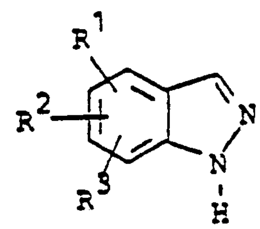

(V),

worin

$R^1$, $R^2$ und $R^3$ gleich oder verschieden sind und die obengenannte Bedeutung haben,

mit Sulfensäurehalogeniden der Formel (VI)

$Z\text{-}S\text{-}CCl_2F$ (VI),

worin

Z für Halogen, bevorzugt für Chlor oder Brom, steht,

in Gegenwart eines Lösungs- und/oder Verdünnungsmittels und in Gegenwart eines Säurebindemittels umsetzt.

Setzt man beispielsweise 5-Trifluormethoxyindazol mit Dichlorfluormethansulfensäurechlorid um, so kann der Reaktionsablauf des erfindungsgemäßen Verfahrens durch das folgende Formelschema wiedergegeben werden:

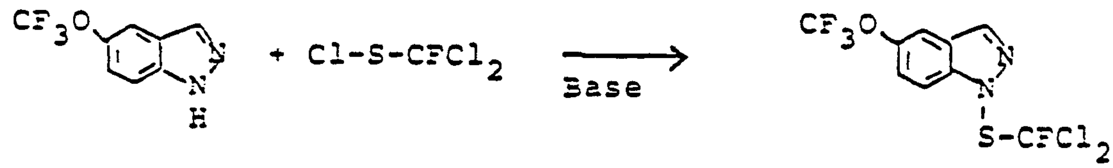


Die bei der Durchführung des erfindungsgemäßen Verfahrens als Ausgangsstoffe eingesetzten Indazole der Formel (V) sind teilweise neu. Sie können nach bekannten Verfahren durch beispielsweise Umsetzung der entsprechenden o-Toluidine mit Alkylnitrit und/oder nitrosen Gasen hergestellt werden (vgl. z. B. DE-OS-2 155 545 und US-3 988 347).

Die für die Herstellung der einzusetzenden Indazole der Formel (V) benötigten o-Toluidine sind ebenfalls teilweise neu und können nach bekannten Verfahren durch Nitrierung der entsprechenden Toluole mit z. B. Nitriersäure und anschließende Reduktion mit Wasserstoff in Gegenwart eines Katalysators wie Raney-Nickel oder Palladium gegebenenfalls auf Aktivkohle hergestellt werden (vgl. z. B. DE-OS-3 023 328 und 3 135 926).

Als Lösungs- und/oder Verdünnungsmittel kommen für das erfindungsgemäße Verfahren insbesondere in Frage: aromatische Kohlenwasserstoffe, wie Benzol und/oder Toluol; halogenierte Kohlenwasserstoffe, wie Methylenchlorid und/oder Tetrachlorkohlenstoff; Nitrile, wie Acetonitril und/oder Propionitril; Ether, wie Tetrahydrofuran und/oder Dioxan; sowie Ester, wie Essigsäureethylester.

Als Säurebindemittel können für das erfindungsgemäße Verfahren alle üblichen organischen und anorganischen Säurebinder eingesetzt werden. Hierzu gehören vorzugsweise tertiäre Amine, wie Triethylamin oder Pyridin; Alkalihydroxide und/oder Alkalicarbonate, wie Natrium- und/oder Kaliumhydroxid, sowie Natriumhydrogencarbonat und/oder Kaliumcarbonat.

Die Reaktionstemperaturen können bei der Durchführung des erfindungsgemäßen Verfahrens in einem größeren Bereich variiert werden. Im allgemeinen arbeitet man bei Temperaturen von etwa 0 bis 150°C, vorzugsweise bei 20 bis 100°C.

Die Indazole der Formel (V) und die Sulfensäurehalogenide der Formel (VI) werden nach dem erfindungsgemäßen Verfahren vorzugsweise in äquimolaren Mengen eingesetzt. Vorzugsweise setzt man pro Mol Indazol etwa 0,8 bis 3, besonders bevorzugt 0,85 bis 1,5, Mole Sulfensäurehalogenid ein.

Die Isolierung der neuen N-sulfenylierten Indazole der allgemeinen Formel (I) erfolgt in allgemein üblicher Art und Weise durch beispielsweise Versetzen des Reaktionsgemisches mit Wasser, Extraktion mit einem organischen Lösungsmittel, Chromatographie oder Destillation.

Die neuen fluorhaltigen N-sulfenylierten Indazole, insbesondere die N-Trihalogenmethylthio-indazole, zeichnen sich durch breite und starke mikrobizide Wirkung aus, von der auch Algen und Schleimorganismen erfaßt werden. Die erfindungsgemäßen Substanzen eignen sich daher vorzüglich zum Schutz technischer Materialien.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmiermittel und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt

werden, wie Lösungen, Emulsionen, Suspensionen, Pulver, Pasten und Granulate.

Diese können in an sich bekannter Weise hergestellt werden, z. B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, 2-Thiocyanomethylthio-benzthiazol, Phenolderivate, wie 2-Phenylphenol, (2,2'-Dihydroxy-5,5'-dichlor)-diphenylmethan und 3-Methyl-4-chlor-phenol.

Die erfindungsgemäßen fluorhaltigen N-sulfenylierten Indazole sind auch für den Einsatz in Pflanzenschutzmitteln geeignet.

Aufgrund ihrer fungiziden Wirkung können sie im Pflanzenschutz eingesetzt werden zur Bekämpfung von Plasmodiophoromycetes, Oomycetes, Chytridiomycetes, Zygomycetes, Ascomycetes, Basidiomycetes, Deuteromycetes.

Aufgrund ihrer bakteriziden Wirkung können die erfindungsgemäßen Wirkstoffe im Pflanzenschutz eingesetzt werden zur Bekämpfung von Pseudomonadaceae, Rhizobiaceae, Enterobaceteriaceae, Corynebacteriaceae und Streptomycetaceae.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanzen- und Saatgut, und des Bodens.

Außerdem eignen sich die erfindungsgemäßen Indazole zur Bekämpfung von tierischen Schädlingen, insbesondere Insekten und Spinnentiere, die in der Landwirtschaft, in Forsten, im Vorrats- und Materialschutz sowie auf dem Hygienesektor vorkommen.

## Beispiele

### Herstellungsbeispiele

#### Beispiel 1

6-Trifluormethoxy-1-dichlorfluormethylsulfenylindazol

Zu 20,2 g (0,1 Mol) 6-Trifluormethoxy-indazol und 10,1 g (0,1 Mol) Triethylamin in 125 ml Toluol werden bei 80 bis 85°C 16,9 g (0,1 Mol) Fluordichlormethylsulfenchlorid, gelöst in 125 ml Toluol, innerhalb einer Stunde zugetropft. Es wird eine Stunde bei gleicher Temperatur nachgerührt. Nach dem Erkalten wird vom ausgefallenen Triethylamin-Hydrochlorid abgesaugt und mit Toluol nachgewaschen. Das mit der Waschphase vereinigte Filtrat wird zweimal mit Wasser gewaschen. Nach dem Trocknen ($MgSO_4$) destilliert man die flüchtigen Bestandteile im Wasserstrahlvakuum ab. Zur Reinigung wird der ölige Rückstand im Hochvakuum destilliert. Siedepunkt 100°C/0,4 mbar.

Ausbeute: 17,1 g (51 % der Theorie).

Die in der nachfolgenden Tabelle aufgeführten Verbindungen wurden auf analoge Weise hergestellt. Zum Teil wurden die Verbindungen durch Chromatographie über Kieselgel mit Toluol als Laufmittel gereinigt.

| Bsp. Nr. | Verbindung | Festpunkt (°C) | Siedepunkt (°C/mbar) | Ausbeute (%) |
|---|---|---|---|---|
| 2 | 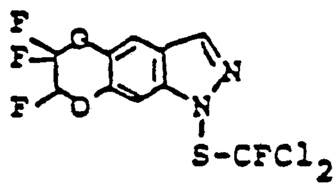 | | 150/0,5 | 41 |
| 3 | 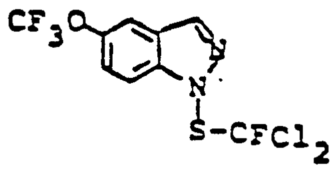 | | 110/0,3 | 75 |
| 4 | 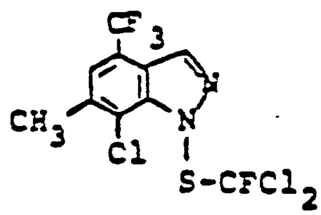 | Fp. 53-55 | | 84 |
| 5 | 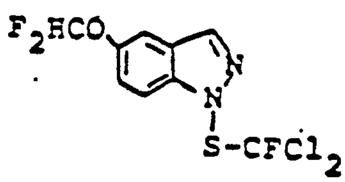 | Öl, $n_D^{20}$: 1,5810 | | 46,5 |
| 6 | 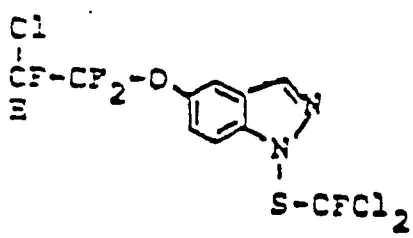 | Sdp. 148-152/0,3 | | 71 |
| 7 | 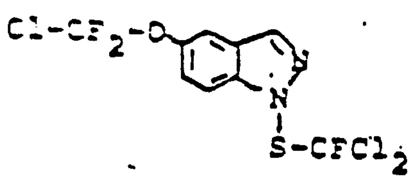 | Sdp. 176-180/0,4 | | 69 |
| 8 | 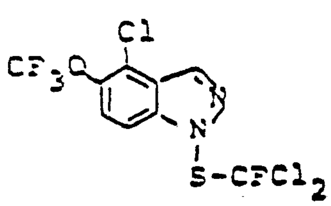 | | Öl $n_D^{20}$: 1,543 | 75 |

| Bsp. Nr. | Verbindung | Festpunkt (°C) | Siedepunkt (°C/mbar) | Ausbeute (%) |
|---|---|---|---|---|
| 9 | 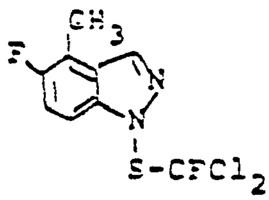 | 120 | | 57 |
| 10 | 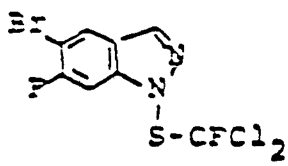 | 72-74 | | 72 |
| 11 | 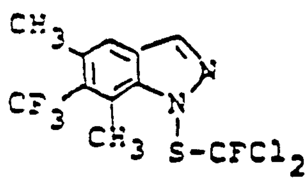 | | Öl $n_D^{20}$: 1,5492 | 58 |
| 12 | 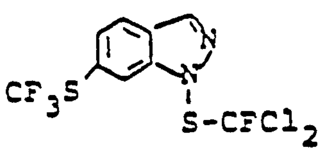 | | Öl $n_D^{20}$: 1,5602 | 85 |
| 13 | 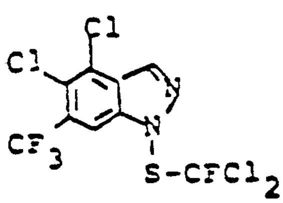 | 90 | | 88 |
| 14 | 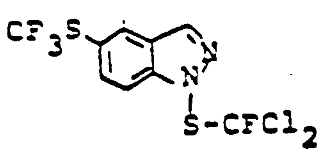 | 54 | | 94 |
| 15 | 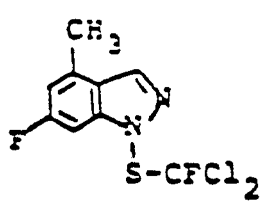 | | Öl $n_D^{20}$: 1,5730 | 78 |

| Bsp. Nr. | Verbindung | Festpunkt (°C) | Siedepunkt (°C/mbar) | Ausbeute (%) |
|---|---|---|---|---|
| 16 | 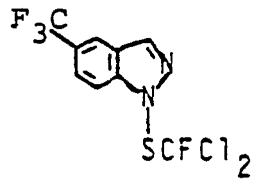 | 42 | | 93 |

## Anwendungsbeispiele

### Beispiel 17

Zum Nachweis der Wirksamkeit gegen Pilze werden die minimalen Hemm-Konzentrationen (MHK) von erfindungsgemäßen Wirkstoffen bestimmt:
Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

**Tabelle 1:** MHK's in mg/l bei der Einwirkung erfindungsgemäßer Substanzen auf Pilze

| | Verbindung der Beispiele Nr. | | | | | | | | | |
|---|---|---|---|---|---|---|---|---|---|---|
| Testorganismen | 1 | 2 | 4 | 8 | 11 | 12 | 3 | 13 | 14 | 15 |
| Alternaria tenuis | 75 | | | 200 | | | 20 | | | 15 |
| Aspergillus niger | 20 | 150 | 500 | 50 | 50 | <20 | 20 | 500 | <20 | 10 |
| Aureobasidium pullulans | 150 | | | 500 | | | 20 | | | 50 |
| Chaetomium globosum | 2 | 100 | <20 | 20 | <20 | <20 | 20 | <20 | <20 | 10 |
| Cladosporium-Cladosporioides | 20 | | | 20 | | | | | | 5 |
| Lentinus tigrinus | 20 | | | 7,5 | | | 0,1 | | | 0,5 |
| Penicillium glaucum | 2 | 100 | <20 | 20 | <20 | <20 | 20 | <20 | <20 | 20 |
| Polyporus versicolor | | | | | | | 2 | | | |
| Sclerophoma pityophila | 35 | | | 200 | | | 0,75 | | | 15 |
| Trichoderma viride | | | | | | | 50 | | | 500 |

### Beispiel 18

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriummitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

**Tabelle 2**

Algen-abtötende Konzentrationen (mg/l) der unten angegebenen Substanz

| Wirkstoff | abtötende Konzentration in mg/l |
|---|---|
| Verbindung des Beispiels Nr. 3 | 50 |

**Beispiel 19** (Wirkung gegen Schleimorganismen)

Erfindungsgemäße Substanzen werden in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid mit 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

**Tabelle 3**

MHK-Werte in mg/l bei der Einwirkung der unten angegebenen Substanz auf Schleimorganismen

| Wirkstoff | MHK in mg/l |
|---|---|
| Verbindung des Beispiels Nr. 3 | 5 - 10 |

**Beispiel 20**

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

**Tabelle**

Drechslera graminea-Test (Gerste)/Saatgutbehandlung (syn. Helminthosporium gramineum)

| Wirkstoff | Wirkstoffaufwandmenge in mg/kg Saatgut | kranke Pflanzen in % der insgesamt aufgelaufenen Pflanzen |
|---|---|---|
| ungebeizt | - | 23,4 |
| <u>bekannt:</u><br>$CH_2$-NH-CS-S<br>\| Zn (Zineb)<br>$CH_2$-NH-CS-S | 500 | 18,0 |
| <u>erfindungsgemäß:</u> | | |
| Verbindung des Beispiels Nr. 1<br>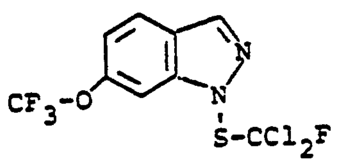 | 500 | 4,1 |

**Patentansprüche**

1. Fluorhaltige N-sulfenylierte Indazole der Formel

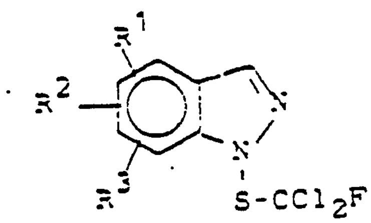

worin

$R^1$, $R^2$ und $R^3$ gleich oder verschieden sind und für Wasserstoff, Fluor, Chlor, Brom, eine Nitrogruppe, eine gegebenenfalls ein- oder mehrfach durch Fluor, Chlor oder Brom substituierte ($C_1$-$C_6$) Alkyl-, ($C_1$-$C_4$) Alkoxy- oder ($C_1$-$C_4$) Alkylthiogruppe stehen oder zwei der Substituenten $R^1$ bis $R^3$ zusammen einen 5,6-Dioxymethylen-, 5,6-Dioxy-difluormethylen-, 5,6-Dioxy-chlorfluormethylen-, 5,6-Dioxyethylen-, 5,6-Dioxy-difluorethylen-, 5,6-Dioxy-trifluorethylen-, 5,6-Dioxy-tetrafluorethylen-, 5,6 Dioxy-chlortrifluorethylen-, 5,6-Dioxy-dichlordifluorethylen-, 5,6-Dioxy-chlordifluorethylen-, 5,6-Dioxy-chlorfluorethylen- oder 5,6-Dioxy-dichlorfluorethylen-Rest bilden, mit der Maßgabe, daß mindestens einer der Substituenten $R^1$ bis $R^3$ für Fluor steht oder ein fluorsubstituiertes Kohlenstoffatom enthält und daß, wenn einer der Substituenten $R^1$, $R^2$ oder $R^3$ für Fluor steht, höchstens einer der beiden verbleibenden Substituenten Wasserstoff ist.

2. Verfahren zur Herstellung der fluorhaltigen N-sulfenylierten Indazole gemäß Anspruch 1, dadurch gekennzeichnet, daß man Indazole der Formel

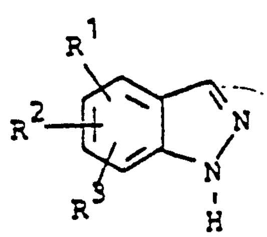

in der

$R^1$, $R^2$ und $R^3$ die in Anspruch 1 angegebene Bedeutung haben, mit Sulfensäurehalogeniden der Formel

Z-$SCCl_2F$

in der

Z für Halogen steht,

in Gegenwart eines Lösungs- und/oder Verdünnungsmittels und in Gegenwart eines Säurebindemittels umsetzt.

3. Verfahren nach Anspruch 2, dadurch gekennzeichnet, daß man als Lösungs- und/oder Verdünnungsmittel aromatische Kohlenwasserstoffe, halogenierte Kohlenwasserstoffe, Nitrile, Ether und/oder Ester einsetzt.

4. Verfahren nach Ansprüchen 2 und 3, dadurch gekennzeichnet, daß man als Säurebindemittel tertiäre Amine, Alkalihydroxide und/oder Alkalicarbonate einsetzt.

5. Mikrobizides Mittel, enthaltend als Wirkstoff fluorhaltige N-sulfenylierte Indazole gemäß Anspruch 1.

6. Mikrobizides Mittel nach Anspruch 5, enthaltend 1 bis 95 % des Wirkstoffs.

7. Verwendung von mikrobiziden Mitteln nach Anspruch 5 um Schutz technischer Materialien vor mikrobieller Veränderung und Zerstörung.

8. Verwendung nach Anspruch 7, dadurch gekennzeichnet, daß man 0.001 bis 5 Gew.-% des Wirkstoffs, bezogen auf des zu schützende Material, einsetzt.

9. Verwendung von mikrobiziden Mitteln nach Anspruch 5 zum Schutz von Pflanzen.

## Claims

1. Fluorine-containing N-sulphenylated indazoles of the formula

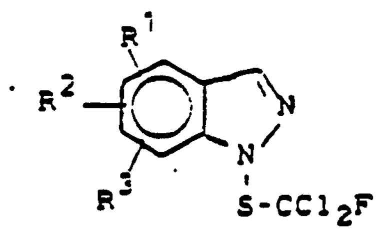

wherein

$R^1$, $R^2$ and $R^3$ are identical or different and stand for hydrogen, fluorine, chlorine, bromine a nitro group, or stand for ($C_1$-$C_6$) alkyl, ($C_1$-$C_4$) alkoxy or ($C_1$-$C_4$) alkylthio group, each of which is optionally substituted once or more than once by fluorine, chlorine or bromine, or two of the substituents $R^1$ to $R^3$ together form a 5,6-dioxymethylene, 5,6-dioxy-difluoromethylene, 5,6-dioxy-chlorofluoromethylene, 5,6-dioxyethylene, 5,6-dioxy-difluoroethylene, 5,6-dioxy-trifluoroethylene, 5,6-dioxy-tetrafluoroethylene, 5,6-dioxy-chlorotrifluoroethylene, 5,6-dioxy-dichlorodifluoroethylene, 5,6-dioxy-chlorodifluoroethylene, 5,6-dioxy-chlorofluoroethylene or 5,6-dioxy-dichlorofluoroethylene radical,

with the proviso that at least one of the substituents $R^1$ to $R^3$ stands for fluorine or contains a fluorine-substituted carbon atom and with the proviso that, in the event that one of the substituents $R^1$, $R^2$ or $R^3$ stands for fluorine, not more than one of the remaining substituents is hydrogen.

2. Process for the preparation of the fluorine-containing N-sulphenylated indazoles according to Claim 1, characterized in that indazoles of the formula

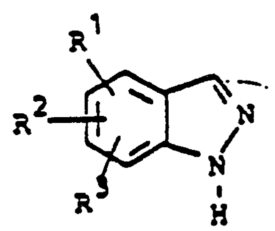

wherein

$R^1$, $R^2$ and $R^3$ have the meaning given in Claim 1,

are reacted, in the presence of a solvent and/or diluent and in the presence of an acid-binding agent, with sulphenyl halides of the formula

$Z\text{-}SCCl_2F$

wherein

Z represents halogen.

3. Process according to Claim 2, characterized in that aromatic hydrocarbons, halogenated hydrocarbons, nitriles, ethers and/or esters are employed as the solvent and/or diluent.

4. Process according to Claims 2 and 3, characterized in that tertiary amines, alkali metal hydroxides and/or alkali metal carbonates are employed as the acid-binding agent.

5. Microbicidal agent containing, as the active compound, fluorine-containing N-sulphenylated indazoles according to Claim 1.

6. Microbicidal agent according to Claim 5, containing 1 to 95 % of the active compound.

7. Use of microbicidal agents according to Claim 5 for protecting industrial materials against microbial change and destruction.

8. Use according to Claim 7, characterized in that 0.001 to 5 % by weight of the active compound, relative to the material to be protected, are employed.

9. Use of microbicidal agents according to Claim 5 for protecting plants.

**Revendications**

1. Indazoles N-sulfénylés fluorés de formule

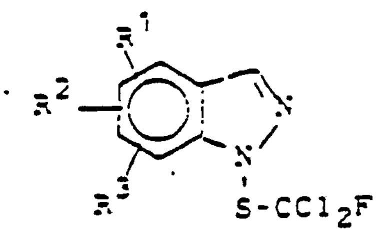

dans laquelle

$R^1$, $R^2$ et $R^3$ sont identiques ou différents et représentent l'hydrogène, le fluor, le chlore, le brome, un groupe nitro, un groupe alkyle en ($C_1$-$C_6$), alcoxy en ($C_1$-$C_4$) ou alkylthio en ($C_1$-$C_4$) éventuellement substitué de façon simple ou multiple par du fluor, du chlore ou du brome, ou deux des substituants $R^1$ à $R^3$ forment ensemble un reste 5,6-dioxyméthylène, 5,6-dioxydifluorométhylène, 5,6-dioxychlorofluorométhylène, 5,6-dioxyéthylène, 5,6-dioxydifluoréthylène, 5,6-dioxytrifluoréthylène, 5,6-dioxytétrafluoréthylène, 5,6-dioxychlorotrifluoréthylène, 5,6-dioxydichlorodifluoréthylène, 5,6-dioxychlorodifluoréthylène, 5,6-dioxychlorofluoréthylène ou 5,6-dioxydichlorofluoréthylène, étant entendu qu'au moins un des substituants $R^1$ à $R^3$ est le fluor ou contient un atome de carbone substitué par le fluor et que, lorsqu'un des substituants $R^1$, $R^2$ ou $R^3$ représente le fluor, un au plus des deux autres substituants est l'hydrogène.

2. Procédé pour la fabrication d'indazoles N-sulfénylés fluorés selon la revendication 1, caractérisé en ce que l'on fait réagir des indazoles de formule

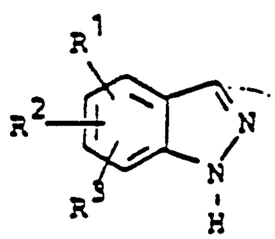

dans laquelle

$R^1$, $R^2$ et $R^3$ ont les significations indiquées dans la revendication 1, avec des halogénures d'acide sulfénique de formule

$Z\text{-}SCCl_2F$

dans laquelle

Z représente un halogène,

en présence d'un solvant et/ou d'un diluant et en présence d'un fixateur d'acide.

3. Procédé selon la revendication 2, caractérisé en ce qu'on utilise comme solvant et/ou diluant des hydrocarbures aromatiques, des hydrocarbures halogénés, des nitriles, des éthers et/ou des esters.

4. Procédé selon les revendications 2 et 3, caractérisé en ce qu'on emploie comme fixateur d'acide des amines tertiaires, des hydroxydes alcalins et/ou des carbonates alcalins.

5. Agents microbicides contenant comme substance active de l'indazole N-sulfénylé fluoré selon la revendication 1.

6. Agents microbicides selon la revendication 5, contenant 1 à 95 % de substance active.

7. Utilisation d'agents microbicides selon la revendication 5 pour la protection de matériaux techniques contre l'altération et la destruction d'origine microbienne.

8. Utilisation selon la revendication 7, caractérisée en ce qu'on utilise 0,001 à 5 % en poids de substance active par rapport au matériau à protéger.

9. Utilisation d'agents microbicides selon la revendication 5 pour la protection de plantes.